# EUROPÄISCHE PATENTSCHRIFT

(11) **EP 2 818 196 B1**
(45) Veröffentlichungstag und Bekanntmachung des Hinweises auf die Patenterteilung: **18.04.2018**
(21) Anmeldenummer: 14002074.4
(22) Anmeldetag: 16.06.2014
(51) Int. Cl.: A61M 16/06

(54) **Kopfbänderung zur Positionierung eines Patienten Interface**
Head band for positioning a patient interface
Sangle de tête destinée au positionnement d'une interface de patient

(30) Priorität: 26.06.2013 DE 202013005712 U
(43) Veröffentlichungstag der Anmeldung: 31.12.2014
(73) Patentinhaber: Löwenstein Medical Technology S.A., 2557 Luxembourg (LU)
(72) Erfinder: Frerichs, Arnold, 21614 Buxtehude (DE); Bechtel, Martin, 21423 Winsen / Luhe (DE)
(74) Vertreter: Marx, Thomas

(56) Entgegenhaltungen:
- EP-A1- 2 359 888
- WO-A1-2012/045127
- US-A1- 2007 186 931
- US-A1- 2010 307 502
- US-A1- 2011 265 796
- US-A1- 2012 289 851
- Anonymous: "EUIPO - eSearch", , 30 September 2011 (2011-09-30), XP055272712, Retrieved from the Internet: URL:https://euipo.europa.eu/eSearch/#detai ls/designs/001905324-0009 [retrieved on 2016-05-13]
- Resmed Resmed Resmed ET AL: "Product Catalogue MASKS AND SLEEP THERAPY SYSTEMS", , 31 July 2013 (2013-07-31), XP055368800, Retrieved from the Internet: URL:http://www.resmed.com/ap/dam/documents /articles/bulletins/device-bulletins/10140 57r11_resmed-product-catalogue_anz_eng.pdf [retrieved on 2017-05-03]
- Resmed Corp ET AL: "Hospital Masks Protect every breath for better treatment outcomes Universal Disposable Bilevel Circuits", , 31 December 2012 (2012-12-31), XP055368777, Retrieved from the Internet: URL:http://www.resmed.com/us/dam/documents /products/Mask/hospital-mask-series/produc t-brochure/1011964r3_hospital-mask-series_ product-brochure_amer_eng.pdf [retrieved on 2017-05-03]
- Resmed Resmed Resmed ET AL: "Product Catalogue MASKS AND Sleep THeRApY SYSTeMS", , 30 November 2011 (2011-11-30), XP055369123, Retrieved from the Internet: URL:http://www.resmed.com/ap/dam/documents /articles/bulletins/mask-bulletins/1014057 -ResMedProductCatalogue.pdf [retrieved on 2017-05-03]

## Beschreibung

### Gebiet der Erfindung

Die Erfindung betrifft eine Kopfbänderung zur Positionierung eines Patienten Interface am Kopf des Anwenders.

### Stand der Technik

Patienten Interface dienen dazu, vom Beatmungsgerät bereitgestelltes Atemgas an den Patienten abzugeben. Das Patienten Interface ist typischerweise über einen Atemgasschlauch mit dem Beatmungsgerät verbunden und wird mittels einer Bänderung am Kopf des Anwenders fixiert.

Da das Patienten Interface nächtelang vom Patienten getragen werden muss, sind hohe Anforderungen an den Tragekomfort und die Passform gestellt. Ebenso ist eine hohe Anforderung an eine einfache Handhabung der Bänderung gestellt.

Neben der genauen Passform des Patienten Interface ist die Befestigung und Fixierung am Kopf des Anwenders sehr wichtig um unangenehme Druckstellen und Undichtigkeiten bzw. Leckagen zu vermeiden.

US2010307502 offenbart eine Halteanordnung für eine Kopfbänderung im Bereich eines Patienten Interface, welches als Nasalstopfen ausgeführt ist, wobei genau zwei Halteanordnungen vorgesehen sind und wobei zwei Halteanordnungen durch mechanische Kodierungen unterschiedlich gestaltet sind.

US2007186931 zeigt eine Halteanordnung für eine Kopfbänderung im Bereich eines Patienten Interface, welches für Kinder ausgeführt ist, wobei zumindest zwei Halteanordnungen vorgesehen sind und wobei die Kopfbänderung durch Kennzeichnungen in Form von Beschriftungen, die die unterschiedlichen Größen angeben, verschieden gestaltet sind.

US2012289851 offenbart eine Halteanordnung für eine Kopfbänderung im Bereich eines Patienten Interface, welches der Gewinnung von CO2-Proben dient, wobei zumindest zwei Halteanordnungen vorgesehen sind und wobei die Halteanordnungen durch Kennzeichnungen unterschiedlich gestaltet sind.

EP2359888 offenbart Halteanordnung für eine Kopfbänderung im Bereich eines Patienten Interface wobei viele unterschiedliche Halteanordnungen vorgeschlagen werden und wobei zumindest zwei Halteanordnungen durch Kennzeichnungen unterschiedlich gestaltet sind.

US2011265796 offenbart eine Halteanordnung für eine Kopfbänderung im Bereich eines Patienten Interface, welches als Nasalstopfen ausgeführt ist, wobei genau zwei Halteanordnungen vorgesehen sind die durch mechanische Kodierungen unterschiedlich gestaltet sind.

WO2012045127 beschreibt eine Halteanordnung für eine Kopfbänderung im Bereich eines Patienten Interface, welches als Nasalstopfen ausgeführt ist, wobei genau zwei Halteanordnungen vorgesehen sind und wobei zwei Halteanordnungen durch Kennzeichnungen und/oder Kodierungen in Form von Beschriftungen unterschiedlich gestaltet sind und somit eindeutig zuördenbar sind.

Das EUIPO Design mit der Nummer 001905324-0009 zeigt ein Patienten Interface mit einer Kopfbänderung, die auf der Innen- und Außenseite in unterschiedlichen Grautönen dargestellt ist.

Die Maske "Quattro FX" (Fa. Resmed) offenbart eine Halteanordnung für eine Kopfbänderung im Bereich eines Patienten Interface wobei zumindest vier Halteanordnungen vorgesehen sind und wobei zumindest zwei Halteanordnungen durch Kennzeichnungen und/oder Kodierungen unterschiedlich gestaltet sind und somit eindeutig zuordenbar sind wobei die Halteanordnung eine Mehrzahl von Halteelementen aufweist, die zur Verbindung der Kopfbänderung mit dem Patienten Interface an zumindest vier Kontaktstellen dienen und wobei die Kennzeichnung durch eine gleichartige Farbgebung der entsprechenden Bauteile Bänderungsclip und/oder Adapter und/oder Aufnahme erfolgt ist und wobei als Kodierung die Halteanordnungen für die Hälften oben und unten des Patienten Interface unterschiedlich ausgebildet sind

Die Maske "Mirage FX for Her" (Fa. Resmed) offenbart, dass Innen- und Außenseite der Kopfbänderung in unterschiedlichen Farben ausgeführt sind.

### Kritik am Stand der Technik

Einige Anwender haben Schwierigkeiten ihre Kopfbänderung nach der Reinigung wieder korrekt an die Maske zu applizieren. Oft werden die Anknüpfungspunkte links und rechts, die Seiten oben und unten oder auch die komplette Kopfbänderung innen und außen vertauscht.

Die bisher auf dem Markt verfügbaren Kopfbänderungen sind oftmals nur auf die Fixierung der Maske am Gesicht des Anwenders ausgelegt, ohne dabei aber den Tragekomfort und speziell die Kenntlichmachung zur fehlerfeien Montage der Kopfbänderung an den Schnittstellen zur Maske zu berücksichtigen.

### Erfindung

Diese Erfindung betrifft eine Kopfbänderung für ein Patienten Interface, die eine eindeutige und einfache Montage der Kopfbänderung ermöglicht.

Aufgabe der vorliegenden Erfindung ist es daher, eine Kopfbänderung und vier Halteanordnungen zwischen der Kopfbänderung und dem Patienten Interface derart zu konstruieren, dass eine einfache und intuitive Zuordnung der Kopfbänderung zu dem Patienten Interface möglich ist und eine individuelle Anpassung der Kopfbänderung und des Verlaufs der Kopfbänderung in der oberen Hälfe des Patienten Interface möglich ist.

Die Aufgabe wird durch eine Halteanordnung für eine Kopfbänderung mit den Merkmalen von Anspruch 1 gelöst.

An den unteren Bänderungslaschen der Kopfbänderung sind die Bänderungsclips vormontiert und durch Endstopper, die über die Enden der Bänderungslaschen greifen und dort vernäht, verklebt oder verschweißt sind, unlösbar mit dem Textil verbunden.

Die Bänderungsclips sind vormontiert und durch Endstopper, die nach der Vormontage der Bänderungsclips in eine Öffnung eingesetzt werden, unlösbar mit dem Textil verbunden.

An der Bänderungslasche vormontierte Bänderungsclips sind in dem Adapter am Maskenkörper verrastet.

Die Kennzeichnung von Bänderungsclip und Adapter ist durch Farbkennzeichnung oder durch gleichermaßen geartete Bedruckung oder direkt im Spritzgusswerkzeug durch erhabene oder vertiefte Kennzeichnung (beispielsweise Symbole, Buchstaben, Zahlen) erzeugt.

Die Kennzeichnung von Bänderungsclip und Adapter erfolgt durch eine mechanische Kodierung.

Die Kennzeichnung erfolgt alternativ durch eine gleichartige Farbgebung der entsprechenden Bauteile Bänderungsclip und/oder Adapter und/oder Aufnahme.

Eine einfache Zuordnung der Bänderungslaschen zu den entsprechenden Seiten der Bänderungsaufnahme erfolgt durch eine Kodierung / Kennzeichnung oder durch Farbkennzeichnungen oder durch gleichermaßen geartete Bedruckung oder direkt im Spritzgusswerkzeug durch erhabene oder vertiefte Kennzeichnungen (beispielsweise Symbole, Buchstaben, Zahlen). Eine Farbkennzeichnung der Bänderungslaschen erfolgt über einen farbigen Klettverschluss und / oder farbige Nähte in der gleichen Farbe wie die entsprechende Seite der Bänderungsaufnahme.

Die Klettverschlüsse der Kopfbänderung sind mit einem zum Ende der Bänderungslaschen weisenden Versatz von 10 ± 3 mm zum einfacheren Handling beim Lösen und Fixieren der einzelnen Bänderungslaschen aufgebracht (vernäht, verklebt, verschweißt).

Innen- und Außenseite der Kopfbänderung sind in unterschiedlichen Farben ausgeführt und/oder durch einen außen oder innen angebrachten Aufdruck kenntlich gemacht. Erfindungsgemäß werden auch zumindest zwei alternative Bänderungsaufnahmen für eine Bänderungslasche vorgeschlagen. Die alternativen Bänderungsaufnahmen sind räumlich benachbart an dem Patienten Interface angeordnet und können gleich oder unterschiedlich gestaltet sein. Dies bietet den Vorteil, dass für eine Bänderungslasche zwei alternative, räumlich benachbarte Bänderungsaufnahmen vorgesehen sind, die es dem Patienten ermöglichen einen alternativen Bänderungsverlauf und/oder einen alternativen Kontaktpunkt zwischen Bänderung und Maske zu wählen.

Die Kennzeichnung / Kodierung der Verbindungselemente zwischen Patienten Interface und Kopfbänderung kann somit durch eine farbliche oder geometrische Verwechslungssicherung erfolgen, bei der korrespondierenden Teile gleich gestaltet sind beziehungsweise zueinander passend gestaltet sind und/oder gleichfarbig sind.
Erfindungsgemäß wird eine Kodierung der Verbindungselemente zwischen Patienten Interface und Kopfbänderung beispielsweise durch Farbkodierung gewählt. Dem Patienten wird es durch eine Kodierung ermöglicht, eine leichte Zuordnung der Bänderungslaschen zu den entsprechenden Aufnahmen zu finden.

Die folgenden Figuren zeigen Ausführungsbeispiele einer Kopfbänderung für ein Patienten Interface der einleitenden Art.
Fig.1: Patienten Interface mit Bänderung
Fig.2: Explosionsdarstellung, Kopfbänderung und Verbindung zum Patienten Interface
Fig. 2a: Varianten der Bänderungsaufnahme im Stirnbereich des Patienten Interface
Fig.3: Patienten Interface mit Bänderung, Bänderungsenden im Wangenbereich mit alternativem Stopper
Fig.4: Detail zu Fig. 3, Bänderungsenden im Wangenbereich mit alternativem Stopper
Fig. 5 Detail einer Maske, identische Aufnahmen links und rechts mit unterschiedlichen Bänderungslaschen

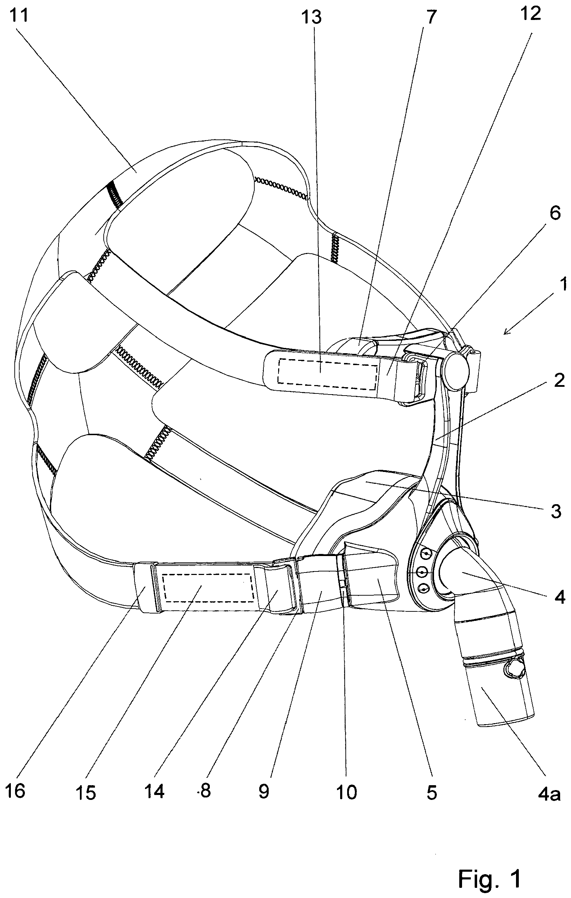

Die Figur 1 zeigt ein Patienten Interface (1) mit angeknüpfter Kopfbänderung (11). Das Patienten Interface (PI) weist einen Maskenkörper (2) auf, an dem über ein Gelenk (4) und eine Drehhülse (4a) der nicht dargestellte Atemgasschlauch mit dem PI verbunden wird. Der Maskenkörper (2) weist als Abdichtung relativ zum Gesicht des Patienten ein Dichtelement (3) in Form eines Maskenwulstes mit einer Lippendichtung auf. Zur Abstützung des Patienten Interface (1) im Bereich der Stirn des Patienten dient eine Stirnabstützung (7). Die Fixierung im Bereich des Kopfes eines Patienten erfolgt über die Kopfbänderung (11). Die oberen Bänderungslaschen (12) werden über Aufnahmen (6) im Stirnbereich mit dem Patienten Interface verknüpft. Die entsprechende Kontaktstelle wird hier gebildet durch die oberen Bänderungslaschen (12) welche durch die Aufnahme (6) geführt werden und mittels Klettverschluss (13) fixiert sind. Die damit geschaffene obere Halteanordnung (19) besteht somit aus Bänderungslasche (12), Aufnahme (6) und Klettverschluss (13). Die obere Halteanordnung (19) kann seitenspezifisch links und rechts unterschiedlich ausgeführt sein; die linke obere Halteanordnung (19') ist vorliegend identisch.
Die unteren Bänderungslaschen (14) werden mittels eines vormontierten Bänderungsclips (8), eines Betätigungselementes (9) und eines Adapters (10) in Aufnahmevorrichtungen (5) im Wangenbereich lösbar an dem Patienten Interface (1) befestigt. Die unteren Bänderungslasche (14) wird mittels Klettverschluss (15) gehalten und durch den Endstopper (16) fixiert. Diese Kontaktstelle bildet somit eine untere Halteanordnung (20) bestehend aus den Elementen: unteren Bänderungslasche (14), Bänderungsclip (8), Betätigungselemente (9), Adapters (10), Klettverschluss (15) und Endstopper (16) sowie Aufnahmevorrichtung (5).
Durch die unterschiedliche Ausgestaltung der oberen Halteanordnung (19) und der unteren Halteanordnung (20) ist eine einfache und eindeutige Zuordnung zu den entsprechenden Aufnahmen (5 und 6) gewährleistet.

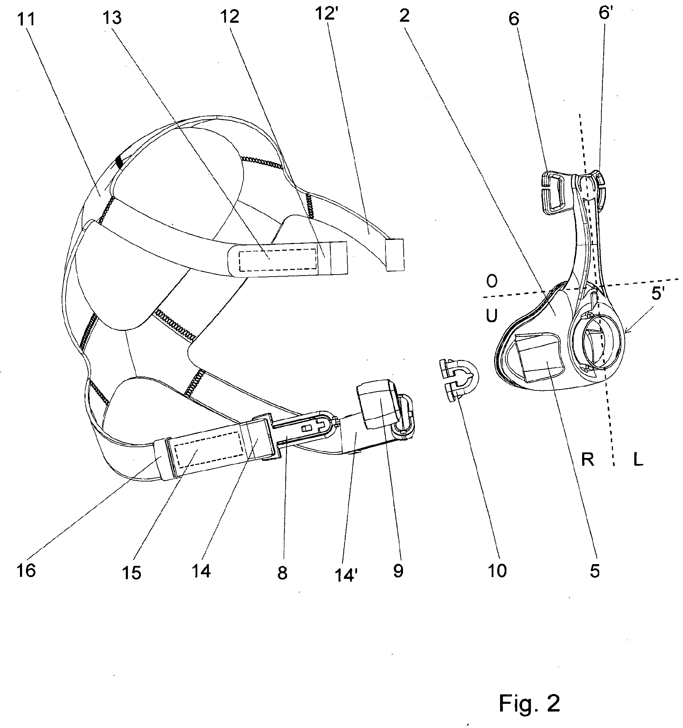

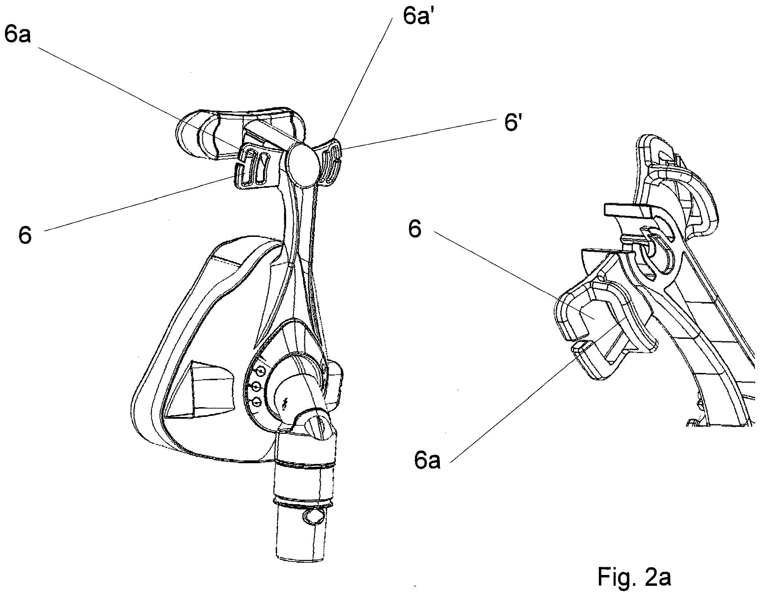

Fig. 2 zeigt in einer Explosionsdarstellung die Einzelteile der Kopfbänderung und die Verbindung zum Patienten Interface. Die Kopfbänderung (11) und das Patienteninterface sind symmetrisch aufgebaut und daher ist eine identische linke (L) und rechte (R) Hälfte vorhanden. Die Verbindung von Kopfbänderung und Patienteninterface ist unten (U) auf der linken (L) und rechten (R) Hälfte identisch ausgeführt (Halteanordnung (20), (20')). Die Verbindung von Kopfbänderung und Patienteninterface ist oben (O) ebenfalls auf der linken (L) und rechten (R) Hälfte identisch (Halteanordnung (20), (20')). Die Verbindung von Kopfbänderung und Patienteninterface oben (O) unterscheidet sich jedoch von der Verbindung von Kopfbänderung und Patienteninterface unten (U). Damit ist eine eindeutige geometrische und/oder farbliche Verwechslungssicherung für die obere (19) und die untere (20) Verbindung gegeben.
Die Kopfbänderung (11) ist aus einem haptisch sehr angenehmen, leicht klettbaren und äußerst atmungsaktiven Material. Die äußere Deckschicht besteht aus Schlaufenmaterial, die Innere aus einem Lycra-Polyester-Gemisch. Innen- und Außenseite der Bänderung (11) sind in unterschiedlichen Farben ausgeführt. Außen kann ein zusätzlicher Aufdruck angebracht sein, der diese Seite als Außenseite kenntlich macht. Innen kann die Bänderung zudem eine andere Farbe ausweisen als außen, um die Innenseite eindeutig kenntlich zu machen. Durch den geringen Materialeinsatz wird ein Wärmeaustausch über den Kopf des Patienten weitestgehend gewährleistet, da große Flächen des Kopfes, speziell des Hinterkopfes unbedeckt bleiben. Durch den optimierten Verlauf der Haltebänder außerhalb der maximalen Ausdehnung des Hinterkopfes entstehen weniger Scherkräfte. Die Geometrie der Bänderung erlaubt die Abdeckung eines weiten Patienten-Anwenderkreises, da unterschiedliche Kopfumfänge von ca. 50 - 60 cm durch nur eine Bänderungsgröße abgedeckt werden können. Die Bänderung ist dreidimensional vorgeformt, dies erleichtert dem Patienten bereits im losen (nicht an die Maske montierten Zustand) der Kopfbänderung eine bessere Zuordnung der Verbindungspunkte zur Maske. Die Klettverschlüsse (13 und 15) der Kopfbänderung (11) sind mit einem zum Bänderungsende der Bänderungslaschen (12, 12', 14, 14') weisenden Versatz von 10 ± 3 mm aufgebracht (vernäht, verklebt, verschweißt), dies ermöglicht dem Patienten ein einfaches Fassen (vereinfachtes Handling) der Lasche zum Lösen und Fixieren der einzelnen Bänder.
Die oberen Bänderungsenden (12, 12') werden von innen durch die Bänderungsaufnahmen (6, 6', 6a, 6a') im Bereich der Stirnabstützung geführt und mit dem Klettverschluss (13) außen auf der Deckschicht aus Schlaufenmaterial fixiert. Die Bänderungsaufnahmen (6, 6') können geschlitzt sein und/oder wie in den Beispielen in Fig. 2a dargestellt, geschlossen sein (6a, 6a'). Die Anordnung von unterschiedlichen Anknüpfpunkten (6, 6`, 6a, 6a`) für die Bänderung bietet dem Patienten verschiedene Geometrien, die unterschiedliche Zugkräfte und unterschiedliche Bänderungsverläufe ermöglichen. Die geschlitzten Bänderungsaufnahmen (6, 6') bietet zusätzlich den Vorteil, dass der Patient, die auf seine Anatomie angepassten, gegeneinander fixierten Bänderungsenden (12, 12') nicht lösen muss, um die Maske abzunehmen. Die Bänderungsenden (12, 12') können durch den Schlitz der Bänderungsaufnahmen (6, 6') gefädelt werden. Die geschlossenen Bänderungsaufnahmen (6a, 6a') bieten dagegen eine Verrutschsicherheit, wenn der Patient beispielsweise die Maske im zusammengefügten Zustand mitsamt der Bänderung über den Kopf stülpen möchte. Dabei könnte es eventuell passieren, dass die Bänderungsenden (12, 12') ungewollt aus den geschlitzten Bänderungsaufnahmen (6, 6') ausfädeln und herausrutschen. Dieses wird durch das Benutzen der geschlossenen Bänderungsaufnahmen (6a, 6a') verhindert. Erfindungsgemäß werden somit auch zumindest zwei alternative Bänderungsaufnahmen (6, 6', 6a, 6a') für eine Bänderungslasche vorgeschlagen. Dies bietet den Vorteil, dass für eine Bänderungslasche (12, 12', 14, 14') zwei alternative, räumlich benachbarte Bänderungsaufnahmen (6, 6', 6a, 6a, 20, 20') vorgesehen sind, die es dem Patienten ermöglichen einen alternativen Bänderungsverlauf und/oder einen alternativen Kontaktpunkt zwischen Bänderung und Maske zu wählen.

Eine einfache Zuordnung der oberen Bänderungslaschen (12, 12') an die entsprechenden Seiten der Bänderungsaufnahmen (6, 6') kann durch eine Kodierung / Kennzeichnung durch Farbkennzeichnungen oder durch gleichermaßen geartete Bedruckung oder direkt im Spritzgusswerkzeug erhabene oder vertiefte Kennzeichnungen erzeugt werden, beispielsweise Symbole, Buchstaben, Zahlen. Eine Farbkennzeichnung kann beispielsweise über einen farbigen Klettverschluss (13) und / oder farbige Nähte erfolgen, die in der gleichen Farbe wie die entsprechende Seite der Bänderungsaufnahmen (6, 6') ausgeführt sind.
An den unteren Bänderungslaschen (14, 14') im Wangenbereich werden die Bänderungsclips (8) vormontiert, das heißt, die Bänderungslasche wird von innen durch die Bänderungsöse des Clips (8) geführt und jeweils mit einem Endstopper (16) versehen, der über das Ende der Bänderungslasche (14) greift und dort vernäht, verklebt oder verschweißt wird. Die Bänderungsclips (8) sind so unlösbar mit dem Textil der Bänderung verbunden, dies gewährleistet eine eindeutige Zuordnung der Kopfbänderung zu den Verbindungspunkten der Maske (hier unten).

Der Adapter (10) für den Bänderungsclip (8) befindet sich in der Aufnahmevorrichtung (5) am Maskenkörper (2) und ist dort leicht schwenkbar verrastet. Zur Montage der unteren Bänderung wird die Schiebehülse (9) über den Bänderungsclip (8) geschoben. Der Bänderungsclip (8) kann dann in dem Adapter (10) geschoben werden und verrastet dort durch seine Schnapphaken. Ist der Bänderungsclip (8) in dem Adapter (10) verrastet, kann diese Verbindung nicht durch Zugkräfte auf den Bänderungsclip (8) gelöst werden, sondern bietet einen sicheren Halt. Die Schiebehülse (9) dient als Betätigungselement zum Lösen der Schnappverbindung. Zum Lösen der Verbindung muss die Schiebehülse (9) in Richtung der Bänderungsaufnahme des Bänderungsclips (8) geschoben/gezogen werden, was ein indirektes Auslenken der Schnapphaken des Bänderungsclips (8) aus dem Adapter (10) bewirkt.
Die Halteelemente (6, 12, 13,) bilden die obere Halteanordnung (19) die links und rechts identisch ausgeführt ist.
Die Halteelemente (5, 8, 9, 10, 14, 15, 16) bilden die untere Halteanordnung (20) die links und rechts identisch ausgeführt ist.

Um eine falsche Montage auszuschließen, werden Bänderungsclip (8) und/oder Schiebehülse (9) und/oder Adapter (10) und/oder Aufnahme (5) gleichermaßen gekennzeichnet. Die Kennzeichnung kann auf beiden Seiten der Maske (rechts und links) oder einseitig (rechts oder links) erfolgen. Dieses kann durch eine geometrische und/oder farbliche Kennzeichnung erfolgen. Also beispielsweise durch Farbkennzeichnung, durch gleichermaßen geartete Bedruckung oder direkt im Spritzgusswerkzeug durch erhabene oder vertiefte Kennzeichnungen (beispielsweise Symbole, Buchstaben, Zahlen) oder durch mechanische Kodierung. Bevorzugt wird eine einseitige Einfärbung des Bänderungsclips (8) und/oder der Schiebehülse (9) und/oder des Adapters (10) in der gleichen Farbe. Dieses kann transluzent oder opak erfolgen. Alternativ oder ergänzend kann auch die Aufnahme (5) im beispielsweise transparent ausgeführten Maskenkörper (2) die gleiche farbliche Kodierung wie der Bänderungsclip (8) oder die Schiebehülse (9) oder der Adapter (10) erhalten.

Die Kennzeichnung der Verbindung (20) von Bänderung (11) und Patienten Interface (1) im Wangenbereich ist somit rechts und links für Bänderungsclip (8), Schiebehülse (9), Adapter (10) und Aufnahme (5) unterschiedlich oder identisch ausgeführt. Neben der Kennzeichnung durch Farbe oder Symbole kann auch eine mechanische Kodierung zwischen Bänderungsclip (8) und/oder Adapter (10) und/oder Aufnahme (5) erfolgen, wobei Bänderungsclip (8) und Adapter (10) und/oder Aufnahme (5) nur jeweils der gleichen Seite (rechts oder links) zusammen passen.
Eine Kennzeichnung kann ebenfalls beispielsweise über einen farbigen Klettverschluss (15) und / oder farbige Nähte sowie eine Kennzeichnung des Endstoppers (16) in der gleichen Farbe wie die entsprechenden Bänderungsclips (8) und Adapter (10) erfolgen.

So wird eine leichte Zuordnung der Bänderungsenden an die entsprechenden Verbindungspunkte zum Patienten Interface sichergestellt.

Wie die zuvor beschriebene Kennzeichnung für eine 4-Punkt-Bänderung, kann auch die gleiche Kennzeichnung bei einer 2- oder 3-Punk-Bänderung oder 5-Punkt-Bänderung erfolgen.

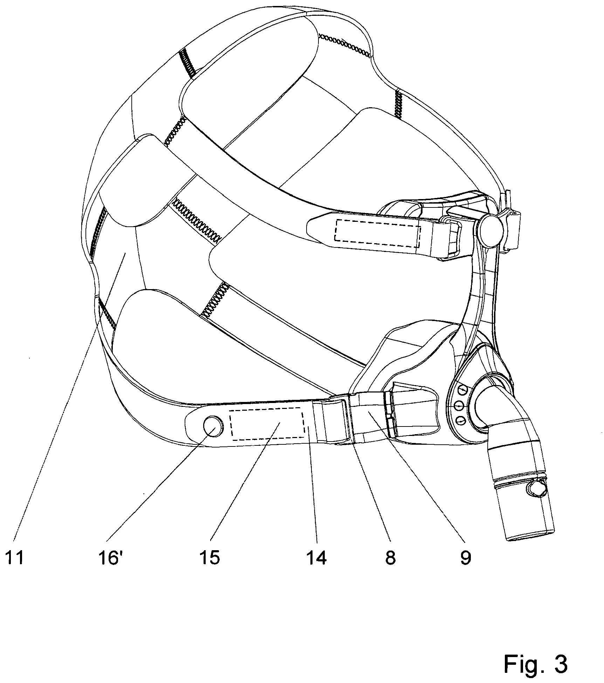

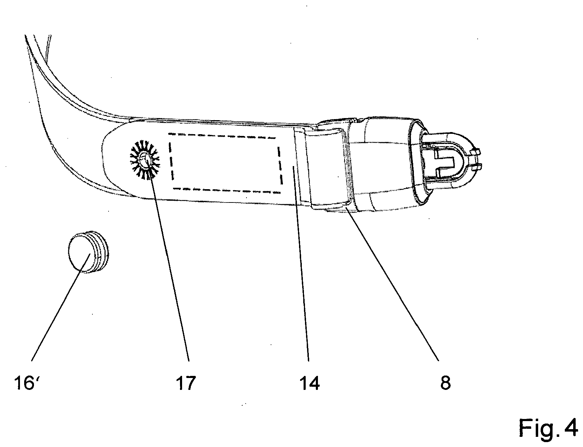

Fig. 3 und 4 zeigen eine weitere Ausführungsform der unteren Bänderungslaschen im Wangenbereich (14) mit der Halteanordnung (20). Die Bänderungsenden sind in dieser Ausführungsform leicht abgerundet ausgeführt und weisen je eine Öffnung (17) auf. Die Öffnung ist durch eine Zick-Zack-Lochnaht verstärkt und befindet sich 14 ± 2 mm vom Ende der Bänderungslaschen. Nach der Vormontage der Bänderungsclips (8) werden die Endstopper (18) in die Öffnungen (17) eingesetzt und verhindern das Herunterrutschen bzw. Verlieren der Bänderclips (8) und gewährleisten ebenfalls eine eindeutige Zuordnung der Kopfbänderung zu den Verbindungspunkten der Maske.
Die Klettverschlüsse (15) der Kopfbänderung (11) sind in diesem Fall mit einem zum Bänderungsende der Bänderungslaschen (14) weisenden Versatz von 22 ± 2 mm aufgebracht (vernäht, verklebt, verschweißt). Die eingesetzten Endstopper (16`) in den Bänderungslaschen (14) ermöglichen dem Patienten ebenfalls ein einfaches Fassen (vereinfachtes Handling) der Lasche zum Lösen und Fixieren der einzelnen Bänder.

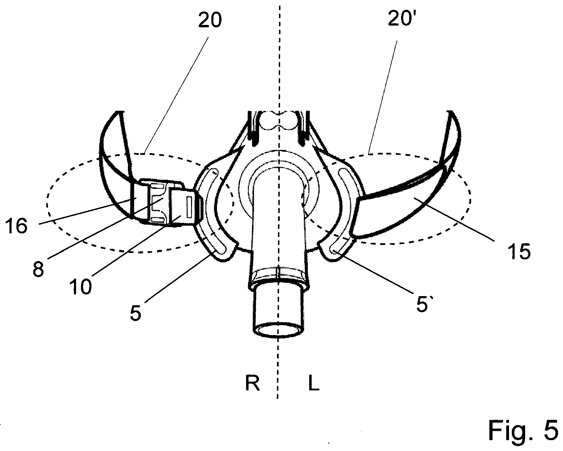

Die Figur 5 zeigt ein Patienten Interface mit angeknüpfter Kopfbänderung. Das Patienten Interface weist einen Maskenkörper mit identischen unteren Aufnahmen (5, 5') auf. Auf der rechten Seite (R) wird die Halteanordnung (20) mittels eines vormontierten Bänderungsclips (8) und eines Adapters (10) in der Aufnahmevorrichtung (5) lösbar an dem Patienten Interface befestigt. Der eingesetzte Endstopper (16) in der Bänderungslasche (14) ermöglicht dem Patienten ein einfaches Fassen der Lasche zum Lösen und Fixieren. Auf der linken Seite (L) wird die Halteanordnung (20') durch die Bänderungslasche gebildet, die von innen durch die Bänderungsaufnahmen (5') geführt und mit dem Klettverschluss (15) außen auf der Deckschicht aus Schlaufenmaterial fixiert wird. Durch die unterschiedliche Ausgestaltung der Halteanordnung links und rechts ist eine einfache Zuordnung zu den entsprechenden Aufnahmen (5 und 5') gewährleistet.

Die Halteelemente (5, 5', 8, 9, 10, 14, 14' 15, 16) bilden die untere Halteanordnung die links (20) und rechts (20') unterschiedlich ausgeführt ist.
Das Patienteninterface ist symmetrisch aufgebaut, daher ist eine identische linke (L) und rechte (R) Hälfte vorhanden. Die Verbindung von Kopfbänderung und Patienteninterface auf der linken (L) Hälfte unterscheidet sich jedoch von der Verbindung von Kopfbänderung und Patienteninterface rechten (R) Hälfte. Damit ist eine eindeutige geometrische und/oder farbliche Verwechslungssicherung für die linke und die rechte Verbindung gegeben.

### Positionsnummern:

- 1: Patient Interface
- 2: Maskenkörper
- 3: Dichtelement
- 4: Schlauchkupplung
- 4a: Drehhülse
- 5: Aufnahmevorrichtung für Bänderung am Maskenkörper,rechts
- 5': Aufnahmevorrichtung für Bänderung am Maskenkörper, links
- 6: Bänderungs-Aufnahme Stirnstütze, rechts
- 6a: Bänderungs-Aufnahme Stirnstütze, Alternative
- 6': Bänderungs-Aufnahme Stirnstütze, links
- 6a': Bänderungs-Aufnahme Stirnstütze, Alternative
- 7: Stirnabstützung
- 8: Bänderungsclip
- 9: Schiebehülse für Bänderungsclip
- 10: Adapter für Bänderungsclip
- 11: Kopfbänderung
- 12: Bänderungslasche Stirnbereich, rechts
- 12': Bänderungslasche Stirnbereich, links
- 13: Klettverschluss
- 14: Bänderungslasche Wangenbereich, rechts
- 14': Bänderungslasche Wangenbereich, links
- 15: Klettverschluss
- 16: Endstopper
- 17: Öffnung für Endstopper
- 18: Endstopper-Knopf
- 19: Halteanordnung oben
- 19': Halteanordnung oben, links
- 20: Halteanordnung unten
- 20': Halteanordnung unten, links

## Patentansprüche

1. Halteanordnung für eine Kopfbänderung (11) im Bereich eines Patienten Interface (1) wobei zumindest vier Halteanordnungen (19, 19', 20, 20') vorgesehen sind und wobei zumindest zwei Halteanordnungen (19, 19', 20, 20') durch Kennzeichnungen und/oder Kodierungen unterschiedlich gestaltet sind und somit eindeutig zuordenbar sind wobei die Halteanordnung eine Mehrzahl von Halteelementen (5, 5', 6, 6', 6a, 6a', 8, 9, 10, 12, 12', 14, 14', 15, 16, 17, 18) aufweist, die zur Verbindung der Kopfbänderung (11) mit dem Patienten Interface (1) an zumindest vier Kontaktstellen dienen und wobei die Kennzeichnung durch eine gleichartige Farbgebung der entsprechenden Bauteile Bänderungsclip (8) und/oder Adapter (10) und/oder Aufnahme (5, 6, 6a) erfolgt ist und wobei als Kodierung die Halteanordnungen (19, 19', 20, 20') für die Hälften oben (O) und unten (U) des Patienten Interface unterschiedlich ausgebildet sind **dadurch gekennzeichnet, dass** Innen- und Außenseite der Kopfbänderung (11) in unterschiedlichen Farben ausgeführt sind und wobei für eine obere Bänderungslasche (12, 12') zwei alternative, räumlich benachbarte obere Bänderungsaufnahmen (6, 6', 6a, 6a) vorgesehen sind, die unterschiedlich gestaltet sind.

2. Halteanordnung nach Anspruch 1 oder 2 **dadurch gekennzeichnet, dass** die Halteanordnungen (19, 19', 20, 20') für die Hälften rechts (R) und links (L) des Patienten Interface unterschiedlich ausgebildet sind.

3. Halteanordnung nach mindestens einem der vorhergehenden Ansprüche **dadurch gekennzeichnet, dass** an den unteren Bänderungslaschen (14) der Kopfbänderung die Bänderungsclip (8) vormontiert und durch Endstopper (16), die über die Enden der Bänderungslaschen (14) greifen und dort vernäht, verklebt oder verschweißt sind, unlösbar mit dem Textil verbunden sind.

4. Halteanordnung nach mindestens einem der vorhergehenden Ansprüche **dadurch gekennzeichnet, dass** an den unteren Bänderungslaschen (14) im Wangenbereich die Bänderungsclips (8) vormontiert und durch Endstopper (18), die nach der Vormontage der Bänderungsclips (8) in eine Öffnung (17) eingesetzt werden, mit dem Textil verbunden.

5. Halteanordnung nach mindestens einem der vorhergehenden Ansprüche **dadurch gekennzeichnet, dass** der an der Bänderungslasche (14) vormontierte Bänderungsclip (8) in dem Adapter (10) am Maskenkörper (2) verrastet.

6. Halteanordnung nach mindestens einem der vorhergehenden Ansprüche **dadurch gekennzeichnet, dass** die Kennzeichnung von Bänderungsclip (8) und Adapter (10) durch Farbkennzeichnung oder durch gleichermaßen geartete Bedruckung oder direkt im Spritzgusswerkzeug durch erhabene oder vertiefte Kennzeichnung erzeugt ist.

7. Halteanordnung nach mindestens einem der vorhergehenden Ansprüche **dadurch gekennzeichnet, dass** die Kennzeichnung von Bänderungsclip (8) und Adapter (10) durch eine mechanische Kodierung erfolgt.

8. Halteanordnung nach mindestens einem der vorhergehenden Ansprüche **dadurch gekennzeichnet, dass** für eine einfache Zuordnung der Bänderungslaschen (12) an die entsprechenden Seiten der Bänderungsaufnahme (6, 6a) eine Kodierung / Kennzeichnung durch Farbkennzeichnungen oder durch gleichermaßen geartete Bedruckung oder direkt im Spritzgusswerkzeug erhabene oder vertiefte Kennzeichnungen erzeugt wird.

9. Halteanordnung nach mindestens einem der vorhergehenden Ansprüche **dadurch gekennzeichnet, dass** eine Farbkennzeichnung über einen farbigen Klettverschluss (13,15) und / oder über ein farbiges Bänderungsende und/oder farbige Nähte in der gleichen Farbe wie die entsprechende Bänderungsaufnahme (5, 6, 6a) erfolgt.

10. Halteanordnung nach mindestens einem der vorhergehenden Ansprüche **dadurch gekennzeichnet, dass** die Klettverschlüsse (13, 15) der Kopfbänderung (11) mit einem zum Ende der Bänderungslaschen (12, 14) weisenden Versatz von 10 ± 3 mm zum einfacheren Handling beim Lösen und Fixieren der einzelnen Bänderungslaschen aufgebracht sind.

## Claims

1. A retainer assembly for a head harness (11) in the region of a patient interface (1), wherein at least four retainer assemblies (19, 19', 20, 20') are provided, and wherein at least two retainer assemblies (19, 19', 20, 20') are designed differently with markings and/or codes and can accordingly be clearly assigned, wherein the retainer assembly has a plurality of retaining elements (5, 5', 6, 6', 6a, 6a', 8, 9, 10, 12, 12', 14, 14', 15, 16, 17, 18) that serve to connect the head harness (11) with the patient interface (1) at at least four contact points, and wherein the marking is provided by an equivalent color of the corresponding harness clip (8) and/or adapter (10) and/or holder (5, 6, 6a) components, and wherein the retainer assemblies (19, 19', 20, 20') are designed differently for the top (O) and bottom (U) halves of the patient interface as code, **characterized in that** the inner and outer side of the head harness (11) are designed in different colors, and wherein two alternative, spatially adjacent top harness holders (6, 6', 6a, 6a') that are designed differently are provided for a top harness strap (12, 12').

2. The retainer assembly according to claim 1 or 2, **characterized in that** the retainer assemblies (19, 19', 20, 20') are designed differently for the right (R) and left (L) halves of the patient interface.

3. The retainer assembly according to at least one of the preceding claims, **characterized in that** the harness clips (8) are pre-mounted on the bottom harness straps (14) of the head harness, and are permanently connected to the textile by end stoppers (16) that grip over the ends of the harness straps (14) where they are sewn, adhered or welded.

4. The retainer assembly according to at least one of the preceding claims, **characterized in that** the harness clips (8) are pre-mounted on the bottom harness straps (14) in the cheek region, and are connected to the textile by end stoppers (18) that are inserted into an opening (17) after pre-installing the harness clips (8).

5. The retainer assembly according to at least one of the preceding claims, **characterized in that** the harness clip (8) pre-installed on the harness strap (14) locks into the adapter (10) on the mask body (2).

6. The retainer assembly according to at least one of the preceding claims, **characterized in that** the marking of the harness clip (8) and adapter (10) is created by a color marking, or by a print of the same kind, or is created directly in the injection mold by an elevated or recessed marking.

7. The retainer assembly according to at least one of the preceding claims, **characterized in that** the harness clip (8) and adapter (10) are marked by a mechanical code.

8. The retainer assembly according to at least one of the preceding claims, **characterized in that** for an easy assignment of the harness straps (12) to the corresponding sides of the harness holder (6, 6a), a code/marking is created by color markings, or by a print of the same kind, or is created directly in the injection mold by elevated or recessed markings.

9. The retainer assembly according to at least one of the preceding claims, **characterized in that** a color marking is created by a colored hook-and-loop fastener (13, 15), and/or by a colored harness end, and/or colored seams in the same color as the corresponding harness holder (5, 6, 6a).

10. The retainer assembly according to at least one of the preceding claims, **characterized in that** the hook-and-loop fasteners (13, 15) of the head harness (11) are applied with an offset of 10 ± 3 mm to the end of the harness straps (12, 14) for easier handling when disconnecting and fixing the individual harness straps.

## Revendications

1. Dispositif de maintien pour un harnais de tête (11) dans la région d'une interface de patient (1), dans lequel il est prévu au moins quatre dispositifs de maintien (19, 19', 20, 20') et dans lequel au moins deux dispositifs de maintien (19, 19', 20, 20') sont réalisés de façon différente par des marquages et/ou des codages et peuvent donc être attribués de façon univoque, le dispositif de maintien présentant une pluralité d'éléments de maintien (5, 5', 6, 6', 6a, 6a', 8, 9, 10, 12, 12', 14, 14', 15, 16, 17, 18), lesquels sont destinés à relier le harnais de tête (11) à l'interface de patient (1) en au moins quatre points de contact, et dans lequel le marquage est réalisé par une coloration similaire des composants correspondants, notamment une attache de harnais (8) et/ou un adaptateur (10) et/ou un logement (5, 6, 6a), et dans lequel, pour le codage, les dispositifs de maintien (19, 19', 20, 20') sont conçus différemment pour les moitiés du haut (O) et du bas (U) de l'interface de patient, **caractérisé en ce que** le côté intérieur et le côté extérieur du harnais de tête (11) sont réalisés dans des couleurs différentes et dans lequel deux logements de harnais supérieurs (6, 6', 6a, 6a) alternatifs adjacents sont prévus pour une patte de harnais supérieure (12, 12'), lesquels sont conçus différemment.

2. Dispositif de maintien selon la revendication 1 ou 2, **caractérisé en ce que** les dispositifs de maintien (19, 19', 20, 20') sont conçus différemment pour les moitiés de droite (R) et de gauche (L) de l'interface de patient.

3. Dispositif de maintien selon l'une au moins des revendications précédentes, **caractérisé en ce que** les attaches de harnais (8) sont prémontées sur les pattes de harnais inférieures (14) du harnais de tête, et reliées au textile de façon inamovible par des butoirs (16) venant en prise par-dessus les extrémités des pattes de harnais (14) et cousus, collés ou soudés de façon inamovible à cet endroit.

4. Dispositif de maintien selon l'une au moins des revendications précédentes, **caractérisé en ce que** les attaches de harnais (8) sont prémontées sur les pattes de harnais inférieures (14) dans la région des joues, et reliées au textile par des butoirs (18) insérés dans une ouverture (17) après le prémontage des attaches de harnais (8).

5. Dispositif de maintien selon l'une au moins des revendications précédentes, **caractérisé en ce que** l'attache de harnais (8) prémontée sur la patte de harnais (14) est encliquetée dans l'adaptateur (10) sur le corps de masque (2).

6. Dispositif de maintien selon l'une au moins des revendications précédentes, **caractérisé en ce que** le marquage de l'attache de harnais (8) et de l'adaptateur (10) est réalisé par un marquage de couleur ou par une impression de type similaire ou directement dans l'outil de moulage par injection par un marquage en relief ou en creux.

7. Dispositif de maintien selon l'une au moins des revendications précédentes, **caractérisé en ce que** le marquage de l'attache de harnais (8) et de l'adaptateur (10) est réalisé par un codage mécanique.

8. Dispositif de maintien selon l'une au moins des revendications précédentes, **caractérisé en ce que** pour une attribution simple des pattes de harnais (12), un codage / marquage est réalisé sur les côtés correspondants du logement de harnais (6, 6a) par des marquages de couleur ou par une impression de type similaire ou directement dans l'outil de moulage par injection par des marquages en relief ou en creux.

9. Dispositif de maintien selon l'une au moins des revendications précédentes, **caractérisé en ce qu'**un marquage de couleur est réalisé par une fermeture velcro colorée (13, 15) et/ou par une extrémité de harnais colorée et/ou par des coutures colorées dans la même couleur que le logement de harnais (5, 6, 6a) correspondant.

10. Dispositif de maintien selon l'une au moins des revendications précédentes, **caractérisé en ce que** les fermetures velcro (13, 15) du harnais de tête (11) sont appliquées avec un décalage de 10 ± 3 mm vers l'extrémité des pattes de harnais (12, 14) pour faciliter la manipulation lors du détachement et de la fixation des différentes pattes de harnais.
